# EUROPEAN PATENT APPLICATION

(11) **EP 1 426 063 A2**
(43) Date of publication of application: **09.06.2004**
(21) Application number: 03029892.1
(22) Date of filing: 11.07.2000
(51) Int. Cl.: A61K 51/12

(54) **Formable integral source material for medical and industrial devices**

(30) Priority: 18.02.2000 US 506611
(62) Divisional of application: 00945323.4
(71) Applicant: Civatech Corporation, Raleigh, NC 27607 (US)
(72) Inventor: O'Foghludha, Fearghus, Durham, NC 27705-5816 (US)
(74) Representative: Horner, Dr. Martin Grenville

(57) **Abstract**

This invention relates to medical and industrial devices, and more particularly to the use of a formable source material having an activatable nuclide in medical and industrial devices.

## Description

### FIELD AND BACKGROUND OF THE INVENTION

The present invention relates to medical and industrial devices, and more particularly to the use of a formable source material having an activatable nuclide in medical and industrial devices.

With respect to medical devices, treatment of various medical conditions, notably cancer, with ionizing radiation is known. In one method of application, sources of gamma (y) radiation, or more recently accelerators producing X-rays or electrons in the approximate energy range of 4 to 20 MeV, are used to direct intense collimated beams of radiation at tumors. To concentrate radiation effects in the tumor while sparing to the greatest possible extent the healthy tissues that usually surround it, multiple cross-firing beams intersecting in the tumor are often used. Because the origin of each beam is commonly at a relatively large distance, e.g., 1 meter, from the tumor, this external beam technique is sometimes referred to as tele- (for distant) therapy. In another method of application, referred to as brachy- (for short) therapy, radioactive sources, encapsulated if necessary to prevent escape of radioactive material into the tissues, are temporarily or permanently implanted in or are closely apposed to the tumor or other target tissue.

Various forms of brachytherapy include interstitial therapy where the sources, sometimes enclosed in needles, tubes or catheters, are physically inserted in the affected tissue; intracavitary therapy where source-containing applicators are introduced into naturally-occurring or artificial body cavities; treatment of certain shallow surface lesions by means of externally applied source-bearing containers often described as "molds"; intra- or endo-arterial irradiation where radioactive sources are inserted in the interior (lumen) of the affected blood vessels, and combinations or variations of these techniques. The type of source used depends on the particular field of application. Some sources emitting β-radiation only, as examples, can be used for intra-arterial brachytherapy because the targeted region of the arterial wall, which is in contact with the source, is heavily irradiated while tissues farther away than the β-ray range receive little or no dose; furthermore, intra-arterial lesions are usually thin, and their deeper regions can be adequately treated even with relatively non-penetrating β-particles. β-sources on the other hand are less suitable than γ-ray sources for irradiation of substantial tumor volumes because large numbers of β-sources must be precisely positioned to ensure that the tumor region is not underdosed if the sources are too far apart nor overdosed if they are too close together.

In interstitial brachytherapy of tumors, which are usually larger than intra-arterial lesions, a wide variety of sources and application methods has been used. The sources and methods of using them are generally familiar to those skilled in the art and include, but are not limited to, radium 226 in equilibrium with its decay products, radon 222, and more recently cobalt 60, cesium 137, gold 198, iridium 192, tantalum 182, iodine 125, palladium 103, and equilibrium mixtures of the β-emitters strontium 90 and yttrium 90, or yttrium 90 alone for specialized sites such as the pituitary gland. The choice of brachytherapy radionuclide will be within the capability of persons skilled in the art and will depend among other factors on the penetrating ability required of the radiation, the radiation emission rate, the half-life, the available activity, the tumor size, shape, location and cellular composition, on the clinical stage to which the tumor has progressed, on the patient's physical condition, on the experience and skill of the operator, on the availability and ability of assisting staff such as medical physicists and dosimetrists, and most particularly on whether the planned implant is to be temporary or permanent.

Specifically, intra-arterial brachytherapy can be used to prevent regrowth of tissue following the treatment of arteries for occlusive diseases, such as atherosclerosis. A commonly used method for treating atherosclerosis is Percutaneous Transluminal Coronary Angioplasty (PTCA). PTCA includes insertion of a balloon catheter through an incision in the femoral artery near the groin, advancement of the balloon over the aortic arch, further advancement within the selected coronary artery, continuing until the balloon portion is placed across the narrowed (stenosed) region. The balloon is inflated, widening the stenosed region of the vessel.

After catheter withdrawal, significant vessel reclosure known as "restenosis" may develop and largely negate the dilation treatment. The reclosure may occur within hours or days of dilation, but more commonly occurs progressively, within six months of the angioplasty. One approach to dealing with restenosis utilizes stents, which are short tubular sections having a lumen therethrough, placed across the recently dilated vessel region. Stents can be either self-expanding or balloon-expandable. Stents are normally left in place indefinitely, though some versions may be designed for eventual removal. Stents, as any permanently implanted device, may cause long term problems. Because the stent constantly pushes radially outward against the vessel wall, the wall may be adversely affected over long periods, particularly in the regions adjoining the ends of the stent. The use of radiation, delivered to the target region either by external i.e., teletherapeutic means or by use of stents which have been made radioactive, has been suggested, for example by U.S. Patent Nos. 5,059,166 to Fischell et al. and 5,199,939 to Dake et al. Suitable active stents can be made e.g. by immersing an inactive metal stent coated with a chelating agent in a solution of the desired radioactive material (see, for example, U.S. Patent No. 5,871,436 to Eury), before insertion in the vascular lumen. Alternatively, the coated stent is immersed in a solution of an unactivated form of the desired nuclide, after which the stent, now bearing inactive material bonded to the metal by the chelator, is exposed to a suitable activating radiation and thereafter inserted in the artery. In yet another method (referred to as ion implantation) of manufacturing radioactive stents, ions of the inactive precursor of the desired radionuclide are driven into the stent surface by means of a high-energy accelerator, after which the stent is activated and inserted in the blood vessel. U.S. Patent No. 5,873,811 to Wang et al. proposes using an adhesive which includes a radioactive material; the adhesive being applied to the vessel wall region to be treated.

These and other prior art stents have a number of limitations. A primary concern is the potential for leakage of the nuclide from the stent into the blood flowing in the vessel. Avoidance of the release of activated nuclide is often dependent on the quality of joining between the metal surfaces of the stent and the nuclide.

Therefore, an object of the present invention is to provide medical and industrial devices having activatable nuclides that are manufactured from materials that are both inexpensively available and easily configured into such devices.

Yet another object of the present invention is to provide devices whose manufacture and use is less hazardous than conventional radioactive devices. There will be less personnel exposure during manufacture because the device is formed before activation, and there is less chance of contamination during use because the radionuclides are integrally bound to the material of the device.

### SUMMARY OF THE INVENTION

These objects and other objects and advantages are provided by the devices and methods of the present invention. The present invention provides an integral source material having at least one nuclide that is activatable by exposure to ionizing radiation. The nuclide is a chemically bound constituent of a polymer making up the formable integral source material. Thus, the integral source material can be configured before activation into a medical or industrial device or component. Suitable medical devices that can be configured can include, but are not limited to the following: stents, seeds, catheters, irradiator tubes, applicators and molds, enclosures, shrouds, ribbons, rods, beads, needles, obturators, discs and the like, and various combinations thereof. Suitable industrial devices include test-objects, large area sources, radioactive enclosures, flood sources, nuclear imaging devices, shrouds and excitation sources for energy-dispersive fluorescence analysis.

### BRIEF DESCRIPTION OF THE DRAWINGS

**FIG. 1** is a perspective view of a typical stent of the present invention.
**FIG. 2** is a perspective view of an embodiment of the present invention.
**FIGS. 3a, 3b and 3c** are side views of typical brachytherapy seeds of the present invention.
**FIG. 4** is a perspective view of another embodiment of the present invention.

### DETAILED DESCRIPTION OF THE INVENTION

The present invention now will be described more fully hereinafter with reference to the accompanying drawings, in which preferred embodiments of the invention are shown. This invention may, however, be embodied in many different forms and should not be construed as limited to the embodiments set forth herein; rather, these embodiments are provided so that this disclosure will be thorough and complete, and will fully convey the scope of the invention to those skilled in the art. Like numbers refer to like elements throughout.

As discussed above, in one aspect the present invention provides an integral source material having at least one nuclide that is activatable by exposure to ionizing radiation. The nuclide is a chemically bound constituent of a polymer making up the integral source material.

Any activatable nuclide that can be bound to a polymer is suitable. In general, the nuclide is preferably located in the backbone of the polymer, although side chain configurations are possible. Without being bound by any one theory, it is believed that the backbone configuration results in a more stable integral source material. Typical weight fractions of nuclide in the polymer will be about 1 to 10 percent, although the selection of higher or lower weight fractions will be within the skill of one in the art.

Radiations usable for activation include neutrons and charged particles, for example, protons, deuterons, alpha particles, etc., the selection of which will be within the skill of one in the art. Once activated, the nuclides preferably emit β or γ radiation or x-rays or combinations thereof. The attainable activity can range from microcuries (µCi) to millicuries (mCi). The activity reached depends on the nature and isotopic abundance of the target nuclide, its activation cross-section, the flux of activating radiation, the irradiation time, half-life and certain other properties.

As stated above, various nuclides that can become a chemically bound constituent of any polymer can be used. Suitable nuclides include, but are not limited to the following: Li, Na, C, F, Al, P, S, Cl, Ca, Ti, V, Cr, Mn, Fe, Co, Ni, Cu, Zn, Ge, Sr, Y, Zr, Mo, Tc, Rh, Pd; I, Cs, Ba, La, Ce, Eu, Gd, Re, Ir, Au, Hg, Pb, Bi, Po and Am. Combinations of such nuclides can be used, for example, a nuclide with a short half life may be used in combination with one having a longer half life. Suitable polymers include, but are not limited to the following: polypropylene, polyethylene terephthalate, nylon, acrylates, polyurethane, polyphenylene oxide blends, polyphenylsulfone, polysulfone, polyether sulfone, polyphenylene sulfide, phenyletheretherketone, polyetherimide, polyphenylmetallosiloxane, fluorine containing polyphosphazines and liquid crystal polymers.

### Medical Devices

The medical devices of the present invention can be utilized in various brachytherapy techniques including as examples angioplasty and treatment of malignant tissue within the brain, lung, esophagus, trachea, oropharynx, uterus, uterine cervix, biliary ductal system, colon or rectum, the gynecological system, prostate, head and neck, including the pituitary gland. The radiation can be applied internally, or externally in the case of surface lesions, while minimizing exposure of healthy tissue and of health care providers.

Suitable medical devices that can be configured can include, but are not limited to the following: stents, seeds, catheters, irradiator tubes, applicators and molds, enclosures, shrouds, ribbons, rods, beads, needles, obturators, discs, and the like, and various combinations thereof. Devices of almost any shape can be fabricated while the polymer and its chemically bound nuclide are inert i.e., non-activated. importantly, such devices can be formed without radiation hazard using existing polymer processing techniques including the use of plasticizers to modify physical properties such as flexibility. Moreover, such devices may be fabricated to take into account the volume (or area in the case of surface lesions) of the tissue to be irradiated, its location, and the depth of penetration needed.

Intra-arterial stent devices and components produced according to the present invention are generally thin-walled cylindrical structures whose rigid or flexible outer walls (or covering layers affixed to those walls) are formed from a base polymer matrix having at least one activatable target nuclide as a tightly-bound chemical constituent thereof. The stents, after activation, can be placed in blood vessels in order to treat, or aid in preventing, the re-stenosis that often follows initially successful angioplastic dilation.

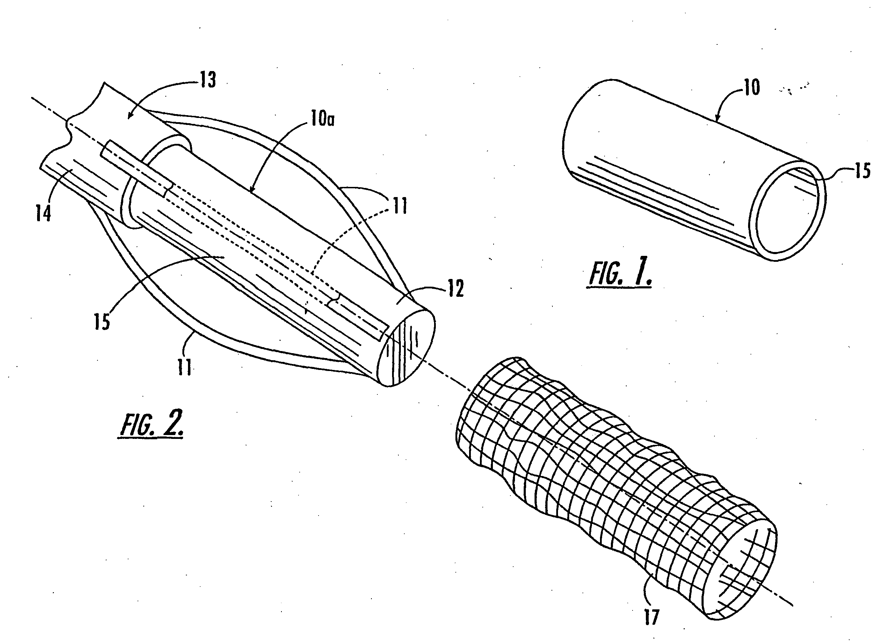

A device of the present invention configured accordingly to the present invention and taking the form of a stent **10** is shown in **FIG. 1.**

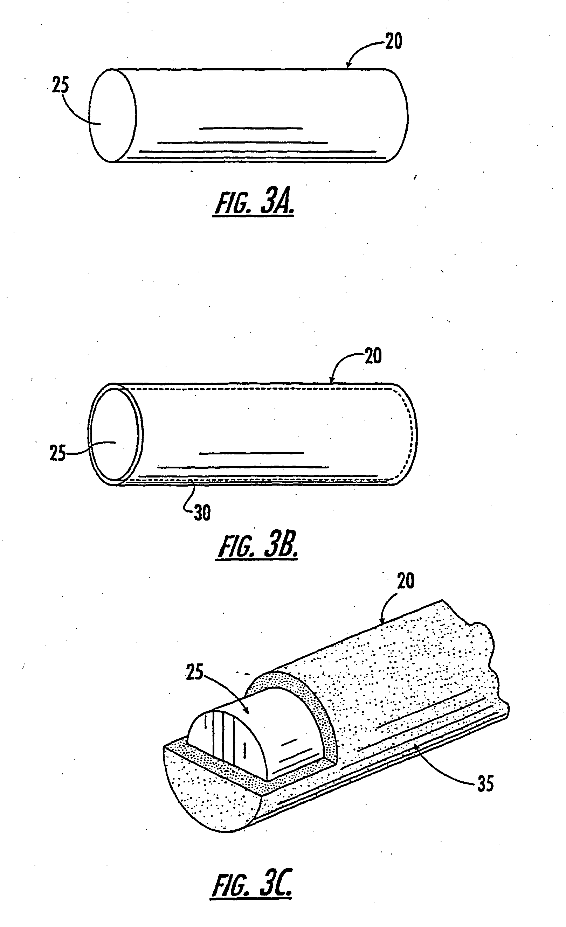

In the example of a radioactive stent device **10a** shown in **FIG. 2,** one end of each of several flexible members **11** (three of which are shown), preferably composed of a polymer rather than a metallic material, are attached at the distal end **12** by a flexible introducing cable **13** in the form of a steerable catheter (which may if desired be furnished with a balloon), the proximal end **14** of which is outside the patient's body. The other end of each flexible member is attached to the tip of a central wire **15** coaxial with and sliding within the introducer proper **13.** Medical staff can retract or advance the tip of the central member by means of an external control, retraction causing a given point on a flexible member to move radially outward and advancement causing a collapse inward. A loosely-woven tubular mesh **17** composed of the integral source material of the invention is attached to the cage-like assembly of flexible members. On operation of the retraction mechanism, the mesh **17** is expanded outwards so that it bears against the vessel wall from which, if desired, it can be slightly separated by a very thin layer of balloon material (not shown). The mesh, the flexibility of whose constituent threads can, if desired, be modified during the manufacturing process by means of plasticizers, can be activated separately from the rest of the device by exposure to a suitable source of radiation, and then attached to the expanding/collapsing cage. Alternatively, the entire end of the device can be made detachable; this permits the mesh to be attached to the expanding mechanism in complete safety while the mesh is still inactive. The entire end with attached mesh is then exposed to the activating radiation and when suitably active is re-connected, an operation that can be carried out more quickly and thus more safely than the more complex one of mounting an already active mesh segment to the flexible members.

In one embodiment of the invention the mesh **17** is fabricated from threads or strands of the base polymer polyarylene ether phosphine oxide which contains as target substance (i.e. activatable nuclide) about 9.7% by weight of phosphorus 31, whose isotopic abundance is 100%. On exposure to thermal neutron radiation the initially inactive phosphorus 31 is activated and becomes phosphorus 32, a pure β-emitter with maximum energy of about 1.72 MeV and half life of about 14 days; none of the other constituents of the base polymer is activated. The phosphorus is substantially chemically contained in the mesh material. Because of the high concentration of phosphorus in the base polymer the material can if desired be activated to a saturation specific activity of about 10 mCi per gram of polymer by exposure to thermal neutron fluence rates that are readily produced by typical nuclear reactors. Charged-particle activating beams from accelerators may be used when the desired radionuclide cannot easily be produced (as ³²P can) by neutron capture.

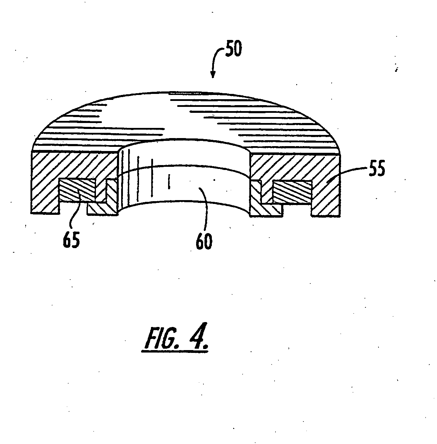

Another exemplary device of the present invention takes the form of a brachytherapy seed **20** shown in **FIGS. 3a, 3b** and **3c.** Such seeds, suitably placed in and around tumors, can deliver high doses to the tumor without unduly adverse effects on neighboring healthy tissues. In one embodiment as shown in **FIG. 3a,** the seed **20** is solid throughout and is essentially formed from the base matrix polymer **25** with activatable nuclide and encapsulation to prevent escape of radioactive material is essentially performed by the invention material itself, which can shed radioactivity only in the form of swarf produced by abrasion. In another embodiment as shown in **FIG. 3b,** the seed **20** has a core of the base matrix polymer **25** with activatable nuclide and an outer layer or sheath **30** of an inert polymer material the same or different from the polymer of the base matrix polymer. Stated otherwise, the active integral source material is encapsulated within an inactive polymer. In yet another embodiment shown in **FIG. 3c,** the seed **20** has a core of the base matrix polymer **25** and an outer layer **30** or sheath of inactive polymer wherein the polymer contains additional screening material **35.** Thus, the outer layer augments the self-encapsulating role of the invention material and simultaneously filters the photon output and screens out undesired β-particles. Seeds are preferably nylon or polyethylene and the target nuclides are selected from the group consisting of possible precursors, depending on the activation route, of the desired radioactive species.

Such seeds can be introduced into the tumor through a variety of ways, e.g., via flexible catheter, cannula with trocar point, etc., the selection and use of which will be within the skill of one in the art. Exemplary other seeds are discussed in, for example, U.S. Patent No. 5,405,309 to Garden Jr., U.S. Patent No. 5,354,257 to Roubin, U.S. Patent No. 5,342,283 to Good, U.S. Patent No. 4,891,165 to Suthanthirian, U.S. Patent No. 4,702,228 to Russell et al., U.S. Patent No. 4,323,055 to Kubiatowicz and U.S. Patent No. 3,351,049 to Lawrence, the disclosures of which are incorporated herein by reference in their entirety.

In accordance with well-known brachytherapy techniques such seeds, often arranged in linear strands or in more complex arrays, can also be inserted in channels or recesses in obturators conformed to fit closely into body cavities such as for example the rectum, vaginal cavity, uterine cervix, nasal cavity, auditory canal, maxillary antrum, etc., with the objective of irradiating lesions located at or just beneath the lining of the cavity into which the obturator is inserted. Placement of seeds on the surface of a mold or plaque is also possible in order to treat lesions located at or immediately below the surface area to which the radiant sources are mounted, usually on a stand-off mold.

The above-described stents and seeds are merely exemplary of the type of devices that can be fabricated. The formability of the source material is highly advantageous since the material can be machined (drilled, milled, turned, sawn, etc.), molded, tested and the like. The selection of the fabrication method (which can include micromachining with laser radiation) will be within the skill of one in the art, to make a myriad of devices. Other types of devices include tubing designed to irradiate liquids as they pass through it, for example in blood irradiators. Although such tubing can be made by extruding already active material, this method is hazardous and unavoidably contaminates the forming devices. The integral source of the present invention can be used to extrude inert forms of subsequently activated polymers; this method is safe and eliminates radiation contamination of the forming equipment.

Using the integral source material of the invention, the above interstitial, intercavitary, and surface procedures can be carried out with the advantage, among others, that the radiation emission need no longer be confined to discrete seed, tube or needle locations but can be arranged, because of the formability of the source material, in spatial patterns whose activity can vary continuously over the treated area; an additional advantage is that the flexibility of the invention source materials enables them to be used in the form of long uniformly-activated flexible strands which can be bent or curved at will, rather than in the necessarily straight-line configurations of a source composed of rigid segments. The continuous-strand source, in addition to its flexibility, has the advantage that the nested surfaces surrounding the source, over any one of which the radiation intensity is constant (i.e., the isodose surfaces) do not show depressions or pinches, as do the surfaces surrounding strands made up of multiple seeds interrupted by gaps, considerably facilitating the specification, calculation and measurement of the delivered dose. The typically lower mass density and atomic number of the typical invention seeds also diminishes the known phenomenon of flux anisotropy, that is the occurrence of smaller fluxes opposite the ends of a seed relative to the fluxes observed laterally, a troublesome dosimetric complication that is exacerbated when multiple seeds are used, e.g. as an array of line sources, each line composed of seeds having inactive gaps between them. The single-seed anisotropy is caused in part by the different degrees of intra-source absorption as radiation seeks to escape along the seed axis or in a direction perpendicular to that axis; anisotropy is further exaggerated when, as in most presently available seeds, a metal encapsulation is used, usually thicker at the ends of the seed than in its lateral wall and usually composed of higher density and higher atomic number material than that used in the seeds of the invention.

As an example, it is known that a radial intensity pattern can be produced that is approximately but not totally uniform over the plane of, e.g., a roughly circular lesion, when the lesion is separated by a calculable distance from a second plane, approximately parallel to the first in which radiating material is arranged in the form of a ring of seeds, short tubes, or other discrete sources. Because the ring consists of several discrete sources rather than a single continuous one, as field calculations assume, the intensity distribution in the plane of the lesion is never perfectly uniform. Use of continuous strand sources, with no activity gaps, minimizes this difficulty. In addition, when the radius of the lesion is large, it becomes necessary to add a second ring and in extreme cases a point-like central source to combat the fall of intensity at the center of the arrangement. Using the formable materials of the invention, a compensatory activity gradient can be produced by machining a block of inert, i.e., not yet activated source material, into the form of a lens, thereby adjusting the mass and therefore the activity immediately above a specific point of the lesion in such a way that, in combination with radiation emitted from all other regions, the doses delivered at all points in the plane of the lesion are the same or very nearly so.

### Industrial Devices

Some examples of industrial devices that can be fabricated by means of the invention are:
(a) Test-objects. Test objects, such as one example, a miniature checkerboard, consisting of alternating active and inactive squares, are easily made from invention materials. The squares, the proposed active ones of which contain target nuclides while the proposed inactive ones do not, are assembled while still inactive, i.e. without hazard, and the whole is then exposed to the activating agent, resulting in a checkerboard or other complex-patterned test-object which can be used to determine the resolving power of autoradiographic emulsions or other high spatial resolution detectors. Larger objects, e.g. bar-charts or similar test objects, can be fabricated for calibration, e.g., of gamma cameras.
(b) Large area sources. In some thickness gauging applications, for example in automatic on-line quality control in the manufacture of sheeting or textile fabrics, rectangular sources are often used whose longer sides equal or exceed the width (often considerable) of the material under test; those sources that rely on beta-emitters, whether of the pure beta or beta-gamma type, present a difficult encapsulation problem because the sealing must be rugged enough to resist puncture in an industrial environment but must be simultaneously thin enough to permit adequate passage of the particles whose transmission through the material being inspected is a measure of absorber thickness. Suitable sources are easily fabricated from slabs of unactivated integral source material fabricated to the proper dimensions by molding or machining, after which the target atoms are activated by exposure to neutrons or other activating fluxes. This relatively safe and convenient operation is in contrast with the conventional method of manufacture in which an already radioactive and thus hazardous material must first be uniformly distributed on and then uniformly affixed to a substrate of the required shape before encapsulation in situ. Using invention materials, the discs can be encapsulated before activation by dipping in or otherwise coating with a non-activatable polymer.
(c) Radioactive Enclosures. Invention materials may be used to fabricate enclosures with radioactive walls as a means of irradiating target objects placed within the enclosures. Depending on the nature (β, γ or (β + γ)) of the radiations emitted from the walls, the phase (i.e., sold or liquid) and the dimensions and absorbing properties of the targets, the enclosure can be in the form of rigid or flexible tubing of internal diameters as small as a few mm, or in shapes which tightly invest objects of quite irregular form such as electronic components, or as spheres, ellipsoids or cylinders of large dimensions (tens of cm). Radioactive tubing made from invention materials may be used, as an example, to irradiate flowing blood in the technique known as human extra-corporeal irradiation (ECI), where blood from a living subject is passed through an external irradiator and is than continuously returned to the subject's vascular circulation. The tubing walls may emit γ-radiation, with the disadvantages that energy-transfer to the flowing blood is small and that the irradiator must be heavily shielded; or alternatively, the wall emission may consist only of β particles, with the advantage that energy transfer is very efficient and shielding requirements are minimal. If nonradioactive tubing is used, as in existing ECI devices, the sources must be outside the tubing, which interposes absorbing material, i.e., the tube wall, between the sources and the target liquid within the tube; use of active tubing made from invention material eliminates this disadvantage. A further use of the invention material in ECI is construction of radiating manifolds to maximize the volume of blood instantaneously present in the irradiator. Increasing the cross-section of the tubing that delivers blood to the device is unsatisfactory because β-rays from the walls then heavily irradiate liquid elements near the walls while elements nearer the axis receive a smaller dose or none at all, depending on the β-energy and the tube diameter. This can be avoided by carrying the blood in a large-diameter tube to the plenum of a manifold where it is divided among multiple radiating channels each of sufficiently small diameter to guarantee adequate energy deposition at their axes.
(d) Flood sources. Flood sources used in nuclear medicine quality control, e.g., in evaluation of gamma-camera responses, are a special type of large area source. They are usually in the form of thin discs, typically from about 30 up to about 60 cm in diameter or rectangles of approximately equivalent areas. The flood sources are sometimes in the form of shallow closed but re-fillable tanks containing a radioactive liquid. This approach guarantees the constancy of the activity per unit volume but has the disadvantage that handling radioactive liquids is hazardous. Flood sources are also made by mechanically admixing a small volume of radioactive solution into a suitably-shaped mass of uncured epoxy resin which is then cured to a solid form. Sources made by admixture of already active material must be checked for non-uniformity before first use. This is because the highly viscous nature of the uncured epoxy hinders uniform mixing. Sources made from invention materials on the other hand have absolutely uniform concentration of active materials provided the flux required to activate the target is uniform over the area of the flood source. However, very large sources may be readily assembled to form a mosaic by juxtaposing a number of segments ("tiles") each of which is smaller than the area over which the activating flux is acceptably constant. The active nuclide in current flood sources is usually ⁵⁷Co, which can be produced in, for example, the reactions: ⁵⁸Ni(γ,p)⁵⁷Co, ⁵⁶Fe(p, γ) ⁵⁷Co, ⁵⁶Fe(d,n) ⁵⁷Co, and ⁵⁵Mn(α2n)⁵⁷Co. These reactions are also usable in fabricating sources for x-ray fluorescence analysis described below.
(e) Nuclear imaging devices. For example, rigid rod-like sources used in measuring line-spread functions by methods known to those in the art may be fabricated from the integral source material of the invention. Yet other uses are the creation of resolving-power test arrays in a grating-like pattern of parallel line sources separated by inactive i.e. non-radiating members; such patterns may be made for autoradiographic measurements in micro- as well as the more usual macro-patterns used in testing nuclear medicine and similar instrumentation.
(f) Radiation curing for protective shrouds. Another use of invention materials is the production of sources useful in the radiation curing (i.e., hardening) of protective shrouds. A shroud is a thin protective layer, usually of a polymeric substance, covering the entire surface of, as one example, an electronic circuit board carrying irregularly shaped resistors, capacitors, chips and the like. Curing to confer hardening is often carried out at very high dose rates, by means of a "bath" electron beam or a similar small-area scanning beam, but even when the object is rotated under the beam, the multiply-reentrant surfaces exercise a complex shielding effect on different parts of the shroud with the result that curing success is spotty. Using invention materials, intimate contact is maintained between the irradiator and the layer that is to be cured, ensuring uniform curing and ensuring a high dose-rate. The technique is advantageous where low-throughput curing of highly valuable objects, such as custom-built circuit boards, is the goal. The largest enclosures, in the form of right circular cylinders of up to 50 cm or thereabouts in diameter, and of approximately the same length, are capable of irradiating the surfaces (if β-emitting invention materials are used) and/or the entire volume (with γ emitters) of e.g., the human body. In these applications, the very large radiating area can be assembled by tiling, as before, with the difference that exact equality of emission at each point on the irradiator surface is not so critically important as making flood sources for gamma cameras, where operators often demand areal constancy to within about ±1%.
(g) Excitation sources for x-ray fluorescence analysis. Another example of the use of integral source material is in fabricating sources for energy-dispersive x-ray fluorescence (XRF) studies. XRF is a technique well known to those in the art for qualitative and quantitative analysis of samples for environmental, forensic and other purposes. The procedure requires the use of an exciting source whose emitted photons excite fluorescent x-rays in the sample under analysis. Energy-dispersive examination of these fluorescent x-rays serves to detect the presence and determine the amounts of various fluorescing species in the sample under examination. The XRF excitation sources are often supplied in either annular or disc geometries. The invention materials can be used to produce both types, with the significant advantage over current sources that the invention material source need not be encapsulated in sealed containers; this difficult process involves the need to weld or otherwise seal already active material in a capsule that has a window (often made of Kapton, beryllium, stainless steel, aluminum or some other material); in addition, the window attenuates the source photons, particularly for low energy sources such as ⁵⁵Fe.

Excitation-source materials containing Co⁵⁷ can be produced by the reactions already listed in section (e) above, using poly(phenylmetallosiloxane)s that contain ⁵⁸Ni, ⁵⁶Fe or ⁵⁵Mn as target materials, while the same or other polymers containing ⁵⁴Fe as target atoms can be activated via the reaction ⁵⁴Fe (n,γ)⁵⁵Fe.

A preferred embodiment of an annular XRF excitation source made of integral source material is indicated in **Fig 4.** A suitable polymer that contains ⁵⁸Ni, ⁵⁶Fe, or ⁵⁴Fe is formed into an annular ring **50** having dimensions typically of the order of 0.5 to 1 inch interior diameter, 0.75 to 1.25 inch outer diameter and thickness 0.1 to 0.3 inch. The forming process takes place prior to irradiation while the integral source material of the invention is nonradioactive. A shielding holder **55** made of tungsten, brass or other material (for shielding in desired dimensions) and a retainer ring **60** are made to accommodate the integral source material ring **35.** The ring **65** is irradiated in a gamma, charged particle, or neutron environment to produce the desired excitation source (⁵⁷Co or ⁵⁵Fe), placed in the shielding holder, and fixed in place with the retainer ring, which can be press fit, glued, welded, or otherwise attached.

The foregoing is illustrative of the present invention and is not to be construed as limiting thereof. Although a few exemplary embodiments of this invention have been described, those skilled in the art will readily appreciate that many modifications are possible in the exemplary embodiments without materially departing from the novel teachings and advantages of this invention. Accordingly, all such modifications are intended to be included within the scope of this invention as defined in the claims. Therefore, it is to be understood that the foregoing is illustrative of the present invention and is not to be construed as limited to the specific embodiments disclosed, and that modifications to the disclosed embodiments, as well as other embodiments, are intended to be included within the scope of the appended claims. The invention is defined by the following claims, with equivalents of the claims to be included therein.

## Claims

1. A method of forming an activatable brachytherapy seed, the method comprising:
forming a solid core of a base polymer, the polymer having constituents including inactive phosphorus 31 provided as a chemically bound constituent on a back bone of the polymer;
after forming the solid core, exposing the polymer of the solid core to thermal neutron radiation; and
activating the inactive phosphorus 31 of the polymer with the thermal neutron radiation to provide active phosphorous 32, the phosphorous 32 being a β-emitter having a maximum energy of about 1.72 MeV and a half life of about 14 days; wherein others of the constituents of the polymer are not activated in the activating step.

2. The method of Claim 1, wherein the polymer is polyarylene ether phosphine oxide.

3. The method of Claim 2, wherein the polymer polyarylene ether phosphine oxide of the solid core includes about 9.7% by weight of the inactive phosphorus 31.

4. The method of Claim 1, wherein the polymer is flexible.

5. The method of Claim 1, wherein activating the inactive phosphorus 31 includes activating the inactive phosphorus 31 to provide active phosphorous 32 to a saturation specific activity of at least about 10 mCi per gram of polymer.

6. The method of Claim 1, further comprising forming an inactive outer layer surrounding the solid core, the inactive outer layer comprising an inert polymer material.

7. The method of Claim 1, further comprising forming an outer layer comprising a screening material that surrounds the solid core and filters the photon output and screens out undesired β-particles.

8. A method of forming an activatable brachytherapy seed, the method comprising:
forming a solid core of a base polymer polyarylene ether phosphine oxide, the polyarylene ether phosphine oxide having constituents including inactive phosphorus 31 provided as a chemically bound constituent on a back bone of the polymer polyarylene ether phosphine oxide;
after forming the solid core, exposing the polymer polyarylene ether phosphine oxide solid core to thermal neutron radiation; and
activating the inactive phosphorus 31 of the polymer polyarylene ether phosphine oxide with the thermal neutron radiation to provide active phosphorous 32, the phosphorous 32 being a β-emitter having a maximum energy of about 1.72 MeV and a half life of about 14 days; wherein others of the constituents of the polymer polyarylene ether phosphine oxide are not activated in the activating step.

9. A method of forming an activatable intra-arterial stent, the intra-arterial stent comprising a cylindrical mesh wall, the method comprising:
forming the cylindrical mesh wall from a plurality of base polymer strands, the polymer strands having constituents including inactive phosphorus 31 provided as a chemically bound constituent on a back bone of the polymer;
after forming the cylindrical mesh wall, exposing the plurality of polymer strands of the cylindrical mesh wall to thermal neutron radiation; and
activating the inactive phosphorus 31 of the polymer with the thermal neutron radiation to provide active phosphorous 32, the phosphorous 32 being a β-emitter having a maximum energy of about 1.72 MeV and a half life of about 14 days; wherein others of the constituents of the polymer are not activated in the activating step.

10. The method of Claim 9, wherein the polymer comprises polymer polyarylene ether phosphine oxide.

11. The method of Claim 10, wherein the forming the cylindrical mesh wall includes forming the polymer polyareylene ether phosphine oxide strands so that the strands include about 9.7% by weight of the inactive phosphorus 31.

12. The method of Claim 10, wherein the polymer polyarylene ether phosphine oxide is flexible.

13. The method of Claim 9, wherein activating the inactive phosphorus 31 includes activating the inactive phosphorus 31 to provide active phosphorous 32 to a saturation specific activity of about 10mCi per gram of polymer.

14. An activatable intra-arterial stent, comprising:
a cylindrical mesh wall comprising a plurality of base polymer strands, the polymer strands having constituents including activatable phosphorus 31 provided as a chemically bound constituent on a back bone of the polymer, the phosphorus 31 being activatable with thermal neutron radiation to provide active phosphorous 32, the phosphorous 32 being a β-emitter having a maximum energy of about 1.72 MeV and a half life of about 14 days.

15. The stent of Claim 14, wherein others of the constituents of the polymer are not activatable by thermal neutron radiation.

16. The stent of Claim 14, wherein the polymer is polyary6lene ether phosphine oxide.

17. The stent of Claim 16, wherein the polymer polyarylene ether phosphine oxide of the polymer strands includes about 9.7% by weight of the inactive phosphorus 31.

18. The stent of Claim 14, wherein the activatable phosphorus 31 is activated to provide active phosphorous 32 to a saturation specific activity of at least about 10mCi per gram of polymer.

19. An activatable brachytherapy seed comprising:
a solid core comprising a base polymer, the polymer having constituents including inactive phosphorus 31 provided as a chemically bound constituent on a back bone of the polymer, the phosphorus 31 being activatable with thermal neutron radiation to provide active phosphorous 32, the phosphorous 32 being a β-emitter having a maximum energy of about 1.72 MeV and a half life of about 14 days.

20. The seed of Claim 19, wherein others of the constituents of the polymer are not activatable by thermal neutron radiation.

21. The seed of Claim 19, wherein the polymer is polyarylene ether phosphine oxide.

22. The seed of Claim 21, wherein the polymer polyarylene ether phosphine oxide of the polymer strands includes about 9.7% by weight of the inactive phosphorus 31.

23. The seed of Claim 19, wherein the activatable phosphorus 31 is activated to provide active phosphorous 32 to a saturation specific activity of at least about 10mCi per gram of polymer.

24. The seed of Claim 19, further comprising an inactive outer layer surrounding the solid core, the inactive outer layer comprising an inert polymer material.

25. The seed of Claim 19, further comprising an outer layer comprising a screening material that surrounds the solid core and filters the photon output and screens out undesired β-particles.
